# EUROPEAN PATENT APPLICATION

(11) **EP 3 811 866 A1**
(43) Date of publication of application: **28.04.2021**
(21) Application number: 19204551.6
(22) Date of filing: 22.10.2019
(51) Int. Cl.: A61B 6/00, H05G 1/44, A61B 6/04

(54) **METHOD FOR ASSESSING A POSITION OF A PATIENT TO AN AUTOMATIC EXPOSURE CONTROL CHAMBER**

(71) Applicant: Koninklijke Philips N.V., 5656 AG Eindhoven (NL)
(72) Inventor: HARDER, Tim Philipp, 5656 AE Eindhoven (NL); BUELOW, Thomas, 5656 AE Eindhoven (NL); YOUNG, Stewart, 5656 AE Eindhoven (NL); VON BERG, Jens, 5656 AE Eindhoven (NL); KROENKE, Sven, 5656 AE Eindhoven (NL); BYSTROV, Daniel, 5656 AE Eindhoven (NL); GOOSSEN, André, 5656 AE Eindhoven (NL)
(74) Representative: Philips Intellectual Property & Standards

(57) **Abstract**

Method for assessing a position of a patient to an automatic exposure control chamber, AEC chamber (11, 12), for a medical exam, wherein
a patient is positioned between an X-ray source and the AEC chamber (11, 12);
comprising the steps:
- acquiring (S10) an X-ray image (32) of at least part of the patient, wherein the AEC chamber is configured for detecting a radiation dose of the X-ray source;
- determining (S20) a position of the AEC chamber (11, 12) with respect to the patient from the acquired X-ray image (32);
- determining (S30) an exam protocol performed on the patient dependent on the medical exam to be performed on the patient and determining an ideal position of the AEC chamber (11, 12) with respect to the patient dependent on the exam protocol, wherein the ideal position relates to a position of the patient relative to the AEC chamber (11, 12), in which the detected radiation dose is reliable for the medical exam; and
- determining (S40) a position deviation of the position of the AEC chamber from the ideal position of the AEC chambers.

## Description

### FIELD OF THE INVENTION

The invention relates to a method for assessing a position of a patient to an automatic exposure control chamber, AEC chamber, for a medical exam, a control unit, an X-ray system, a computer program and a computer readable data-carrier.

### BACKGROUND OF THE INVENTION

During a medical exam of a patient using X-rays, the patient is exposed to X-ray radiation. The radiation tends to be harmful when exceeding a predetermined dose of radiation. Thus, it has to be ensured that the benefits of the radiation, in other words the ability to provide X-ray images, outweighs the negative effects.

Therefore, an X-ray system must be able to determine an appropriate amount of radiation dose required for most routine medical exams automatically. One way of achieving this is the application of automatic exposure control chambers, AEC chambers. These AEC chambers are specific areas of a detector, rapidly reporting the amount of radiation dose received. The patient is thus positioned between the X-ray source and the AEC chambers. In this setup, the assumption is that the AEC chamber is adequately positioned and hence allows an approximation of the appropriate radiation dose. Misaligning the AEC chamber, for example behind bony structures of the patient such as the scapula will invalidate this assumption and lead to overexposure of X-ray radiation to the patient.

### SUMMARY OF THE INVENTION

There may therefore be a need for an improved method for assessing a position of a patient to an automatic exposure control chamber.

The object of the present invention is solved by the subject matter of the independent claims where further embodiments are incorporated in the dependent claims. It should be noted that the following described aspect of the invention equally applies to the control unit, to the X-ray system, to the computer program and to the computer readable medium.

According to an aspect, a method for assessing a position of a patient to an automatic exposure control chamber, AEC chamber, for a medical exam, wherein a patient is positioned between an X-ray source and the AEC chamber comprises the following steps. In a first step, an X-ray image of at least part of the patient is acquired, wherein the AEC chamber is configured for detecting a radiation dose, which correlates to a radiation dose of the X-ray source. In a second step, a position of the AEC chamber with respect to the patient is determined from the acquired X-ray image. In a third step, an exam protocol performed on the patient is determined dependent on the medical exam to be performed on the patient and determining an ideal position of the AEC chamber dependent on the exam protocol, wherein the ideal position relates to a position of the patient relative to the AEC chamber, in which the detected radiation dose is reliable for the medical exam. In a fourth step, a position deviation of the position of the AEC chamber from the ideal position of the AEC chambers is determined.

The term "detected radiation dose", as used herein, refers to a dose of radiation, which is detected by the AEC chamber. The radiation dose indicates a duration of exposure to a radiation and/or an intensity of the radiation. Preferably, the detected radiation is integrated over the duration of exposure to determine the radiation dose. The detected radiation dose thereby correlates to a radiation dose sent out by the X-ray source by a correlation factor. This correlation factor is known for each medical exam wherein the patient is in the ideal position to the AEC chamber. In other words the correlation factor indicates how much of the radiation dose sent out by the X-ray source should be detected by the AEC chamber when the patient is in the ideal position depending on a specific medical exam.

The term "ideal position", as used herein, refers to a position of the patient in view of the AEC chamber, as it is considered ideal for determining a reliable radiation dose by the AEC chamber. This ideal position is preferably predetermined dependent on the medical exam, or in other words the exam protocol, performed on the patient. In other words, when the patient is in an ideal position to the AEC chamber, the AEC chamber can detect an X-ray radiation dose that is a reliable indicator of the X-ray radiation in fact used on the patient. In other words, it is known which radiation dose the AEC chamber detects depending on a specific exam protocol, when the patient is in the ideal position to the AEC chamber. The radiation dose detected by the AEC chamber does not have to be equal to the dose the patient is exposed to, however the radiation that the AEC chamber detects should ideally be correlating to the radiation that the patient is exposed to. In a non-ideal position, for example, parts of the X-ray radiation used on the patient is blocked, for example by bones of the patient, although that would not happen if the patient was in the ideal position. In this case, the detection of the AEC chamber correlates to a radiation dose that is lower than the radiation dose, which was in reality used on the patient. In other words the correlation factor is smaller than expected. Since the radiation dose determined by the AEC chamber is used to determine an upper limit of X-ray radiation used on the patient, it is crucial that the radiation dose determined by the AEC chamber reliably relates to the radiation dose the patient is exposed to. In another example, where depending on the medical exam, it is expected that parts of the AEC chamber are blocked for radiation by bones, a non ideal position of the patient to the AEC chamber might lead to another wrong correlation. In such a case, the detection of the AEC chamber correlates to a radiation dose that is higher than the radiation dose, which was in reality used on the patient. This might lead to an early stopping of radiation of the patient leading to a lower quality X-ray image.

The term "reliable for the medical exam", as used herein, describes that the radiation dose that the AEC chamber detects correctly correlates to the radiation dose, the patient is exposed to. Thus, the radiation for the medical exam can be reliably controlled based on the radiation dose detected by the AEC chamber. In other words, the radiation dose detected by the AEC chamber is reliable for the medical exam, if the correlation factor of the AEC chamber equals an expected correlation factor. The correlation factor indicates how the detected radiation dose relates to the radiation dose sent be the X-ray source. The expected correlation factor indicates how the radiation dose that is expected to be detected by the AEC chamber depending on the medical exam and the ideal position of the patient to the AEC chamber, correlates to the radiation dose sent by the X-ray source.

Preferably, the ideal position is defined in view of the exam protocol performed on the patient. For example, if the lungs of the patient should be X-rayed, an ideal position of the patient to the AEC chamber is known to perform the exam protocol for scanning the lungs. In this case, the lungs of the patient should ideally be positioned in a way that the AEC chamber is aligned to the X-ray source with the lungs of the patient being between the X-ray source and the AEC chamber. In the ideal position, an approximation of the appropriate dose of radiation is improved in particular in view of a misalignment of the AEC chamber behind a bony structure such as the scapula, which will invalidate the assumption and lead to overexposure. Preferably, the ideal position is predefined for each exam protocol.

Preferably, the position of the AEC chamber with respect to the patient is determined based on a geometry between the patient and the AEC chamber. Further preferably, the AEC chamber is part of an X-ray detector. Thus, the position of the AEC chamber with respect to the patient is determined based on a geometry between the patient and the X-ray detector.

Preferably, the AEC chamber is part of an X-ray exposure termination device, which allows to stop applying more radiation dose to the patient as soon as a specific predetermined amount of radiation dose in accordance with a medical exam, or in other words a body part of the patient, has been received. The X-ray exposure termination device preferably comprises a plurality of AEC chambers. The AEC chambers are specific areas of the X-ray exposure termination device configured for rapidly reporting an amount of radiation dose received.

Preferably, the AEC chamber is also called active chamber.

Thus, the position of the patient to an automatic exposure control chamber can be assessed based on the determined position deviation in view of the ideal position for the medical exam.

Thus, an improved method for assessing the position of the patient to an automatic exposure control chamber is provided.

In a preferred embodiment, determining the position deviation comprises the following steps: Segmenting at least an anatomical structure of the patient in the X-ray image thereby determining at least one segmented anatomical structure and determining the position deviation dependent on the at least one segmented anatomical structure.

Preferably, the position of the segmented anatomical structure in the X-ray image is compared to the position of the AEC chamber.

Thus, it can be determined, in how far the radiation dose, measured by the AEC chamber, has penetrated the anatomical structure of the patient. Additionally, it can be determined how the position of the AEC chamber to the anatomical structure differs from an ideal position of the AEC chamber to the anatomical structure. Based on that a position deviation can be concluded.

Thus, an improved method for assessing the position of the patient to an AEC chamber is provided.

In a preferred embodiment, determining the position deviation comprises the following steps. Determining an overlap of the at least one segmented anatomical structure with the AEC chamber and determining the position deviation dependent on the determined overlap.

Preferably, the overlap of the segmented anatomical structure with the AEC chamber is determined based on the position of the anatomical structure in the X-ray image and the position of the AEC chamber in the X-ray image. In other words, the overlap describes an area of the segmented anatomical structure overlapping with the AEC chamber in a radiation direction of the X-ray radiation, in particular from the X-ray source to the AEC chamber. Based on the performed medical method an ideal position of the anatomical structure to the AEC chamber is known. For example, the medical exam comprises scanning the lungs of a patient. In the ideal case, the AEC chamber fully covers the lung to be scanned. Preferably, the overlap is defined by a percentage of the area of the anatomical structure, in this case the lung, overlapping with the AEC chamber. Further preferably, a predetermined threshold indicates, if the overlap is considered sufficient for approximating the radiation dose. Thus, it can be determined if the position of the patient to the AEC chamber is sufficient for the approximation of the radiation dose. For example, if the overlap is below the threshold, it can be assessed that the position of the patient to the AEC chamber is sufficiently off that an assessed radiation dose cannot be trusted in.

Thus, an improved method for assessing the position of the patient to an AEC chamber is provided.

In a preferred embodiment, the at least one anatomical structure of the patient in the X-ray image is segmented by a deep learning network.

The deep learning network is configured for learning to semantically segment the anatomical structure in the X-ray image based on a relatively large amount of X-ray images.

A quality of the semantic segmentation of the anatomical structure in the X-ray image directly correlates to a quality of determination of the subsequent position deviation.

Thus, an improved method for assessing the position of the patient to an AEC chamber is provided.

In a preferred embodiment the method comprises the following steps. Determining deviation evaluation data, indicating the quantity of the deviation, by comparing the position of the AEC chamber with the ideal position of the AEC chamber determining condition data, relating to additional conditions of the specific medical exam and determining a root cause for the position deviation dependent on the deviation evaluation data and the condition data.

Preferably, the deviation evaluation data, which are in particular determined by using an image recognition algorithm on the X-ray image, include a translational shift and/or a rotation of the patient, which is not desired. For example, the deviation evaluation data comprise information how far the patient is shifted to a side to the ideal position of the patient in view of the medical exam. In other words, in a case where the deviation evaluation data indicate that only a right part of the AEC chamber covers the anatomical structure, it can be determined that the patient is shifted to the right from the ideal position, where the whole AEC chamber would be covered by the anatomical structure. It thus can be determined that a position deviation is present that most likely is caused by a shift of the patient to the right in view of the ideal position, which thus can be determined as the root cause for the suboptimal position of the patient to the AEC chamber.

In other words, from the shape and size of the segmented anatomical structure a root cause for the position deviation between the determined position and the ideal position can be determined.

A rotation of the patient is generally harder to detect from the lung shape alone than a translational shift of the patient into a direction. Detecting a rotation of the patient requires a comparison with previous images of the same patient.

Together with the deviation evaluation data, condition data is determined. The condition data can comprise a plurality of factors influencing the medical exam. For example, the condition data comprises an identity of the operator leading the medical exam. In this case, the equipment for executing the method comprises means for determining the identity of the operator, for example by an ID card reader or an ID pin terminal. The deviation evaluation data thus is associated with a specific operator. Consequently, depending on the condition data,

For example, the root cause indicates that a specific operator has the tendency to misplace, or in other words shift, the patient to on side in view of the AEC chamber.

In other words, the root cause indicates a reason, causing the position deviation.

Thus, an improved method for assessing the position of the patient to an AEC chamber is provided.

In a preferred embodiment, the condition data comprise a room, where the medical exam takes place, a time, when the medical exam takes place and/or an identification of an operator, who positions the patient with respect to the AEC chamber.

Including additional data indicating different conditional properties opens up the possibility to find the root cause for the position deviation.

Thus, an improved method for assessing the position of the patient to an AEC chamber is provided.

In a preferred embodiment, the root cause is determined using a statistical analysis of the deviation evaluation data of a plurality of medical exams.

In a continuous improvement scenario, the described method, in particular determining the position deviation and the root cause, is run for an extended amount of images to reach statistical significance for different conditions, for example different operators. This analysis can serve as initial baseline result to be improved in the future.

Preferably, a database with a plurality of X-ray images from a plurality of medical exams is provided, based on which the statistical analysis for determining the root cause is executed.

Thus, an improved method for assessing the position of the patient to an AEC chamber is provided.

In a preferred embodiment the root cause is displayed on a graphical user interface for the operator.

Preferably, the graphic user interface comprises a dashboard for displaying the root cause.

The operator thus is informed about a possible root cause for the position deviation and is sensitized for errors leading to the position deviation. Thus, in future executions of a similar medical exam, the operator does most likely not repeat the detected error.

Thus, an improved method for assessing the position of the patient to an AEC chamber is provided.

In a preferred embodiment, a database of a plurality of root causes associated with a plurality of condition data is provided and a root cause is predicted depending on the condition data and the associated root causes before executing the medical exam.

Preferably, the operator is provided with a predicted root cause for a potential position deviation during the medical exam. For example, over a plurality of medical exams, a specific operator had the tendencies to misplace the patient to on side in view of the ideal position to the AEC chamber, the operator can be provided with this information before the medical exam begins. Thus, the operator is alarmed to pay attention to the position of the patient in a specific way. The condition data for example comprises an identification for a specific X-ray source. In this case, it might be determined over a plurality of medical exams that there is a high chance that the root cause for former position deviations might be a miscalibration of the X-ray source.

Thus, an improved method for assessing the position of the patient to an AEC chamber is provided.

In a preferred embodiment, the exam protocol is determined automatically dependent on the medical exam.

Preferably, the exam protocol is determined based on a type of exam, which further preferably is determined using machine learning or based on a given meta-description of the exam.

In a preferred embodiment, the position deviation is provided to the operator.

Preferably, the position deviation of the specific medical exam is provided to the operator and/or a dose manager responsible for the medical exam. The present position deviation can thus be used to evaluate, if the feedback of the AEC chamber is reliable enough to depend the radiation dose for the medical exam on the AEC chamber.

Preferably, the position deviation is presented to the operator in form of a number on a graphic user interface indicating a misalignment of the patient to the AEC chamber.

According to an aspect of the invention, a control unit is configured for executing a method for positioning of an automatic exposure control chamber, AEC chamber, for a medical exam, as described herein.

According to an aspect of the invention, an X-ray system comprises an X-ray source, configured for radiating a patient for a medical exam with X-ray radiation, an automatic exposure control chamber, AEC chamber, configured for detecting a radiation dose of the X-ray source and a control unit, as described herein.

Preferably, the X-ray system comprises a condition unit, configured for determining condition data relating to additional conditions of the specific medical exam. The X-ray system preferably comprises an identification unit, configured for identifying the operator performing the medical exam. The identification unit for example comprises a terminal for entering an identification number or for reading an identification card, identifying the operator. Further for example, the condition unit comprises a terminal for entering a room identification number, for identifying the room, in which the X-ray system is disposed and the medical exam is performed. Further for example, the condition unit comprises a clock for determining a time, the medical exam is performed.

According to an aspect of the invention, a computer program comprises instructions which, when the program is executed by a computer, cause the computer to carry out the method, as described herein.

According to an aspect of the invention, a computer-readable data carrier is provided having stored there on the computer program, as described herein.

### BRIEF DESCRIPTION OF THE DRAWINGS

Fig 1a shows an X-ray image of a lung in front of an X-ray detector in an ideal position;
Fig 1b shows another X-ray image of a lung in front of a X-ray detector in an unidealposition; and
Fig. 2 shows a schematic diagram of a method for assessing a position of a patient to an automatic exposure control chamber, AEC chamber, for a medical exam.

### DETAILED DESCRIPTION OF EMBODIMENTS

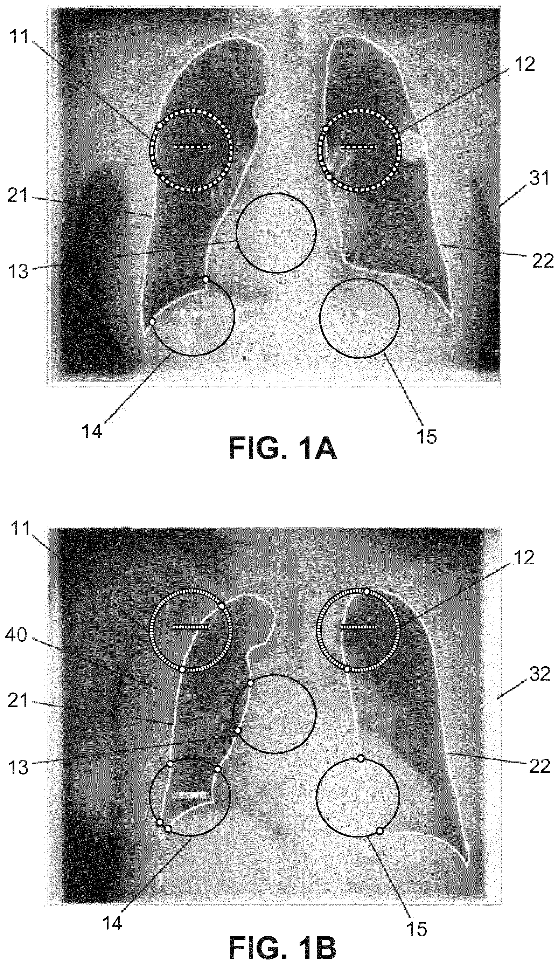

Fig. 1 shows a first X-ray image 31 of a left lung 21 and a right lung 22. The first X-ray image 31 is determined by a medical exam, wherein an exam protocol is followed. Radiological images may be flipped vertically. Depending on that, what is described herein as left lung 21 can in reality be the right lung of the patient and what is described herein as right lung 22 can in reality be the left lung of the patient. A patient is positioned between an X-ray source, configured for radiating the patient with X-ray radiation, and an X-ray detector. The X-ray detector is configured for detecting a radiation dose correlating to the X-ray radiation of the X-ray source. The X-ray detector comprises a plurality of AEC chambers, namely a first AEC chamber 11, a second AEC chamber 12, a third AEC chamber 13, a fourth AEC chamber 14 and a fifth AEC chamber 15.

In this case, the medical exam is to X-ray the lungs 21, 22. Thus, the first AEC chamber 11 and the second AEC chamber 12 are relevant for estimating the radiation dose, the patient has been exposed by the X-ray source during performing the x-ray scan of the lungs 21, 22. Thus, based on the radiation dose received by the first AEC chamber 11 and the second AEC chamber 12, the radiation dose the patient has been exposed to can be estimated. To be specific, the radiation dose detected by the AEC chambers 11, 12 is compared to a radiation dose that is expected to be detected by the AEC chambers 11, 12 depending on the specific medical exam that is performed on the patient. The radiation dose detected by the AEC chambers 11, 12 rather correlates to the radiation dose, the patient is exposed to. Consequently, the X-ray source stops the radiation, if a predetermined threshold for the detected radiation dose has been reached. As can be seen from fig. 1, the patient is ideally positioned in such a way between the radiation source and the AEC chambers, that the first AEC chamber 11 and the second AEC chamber 12 are overlapping the left lung 21 and the right lung 22 respectively as much as possible. In this case, an overlap ratio of the left lung 21 and the right lung 22 with the first AEC chamber 11 and the second AEC chamber 12 is around 90%. Thus, the radiation dose that the first AEC chamber 11 and the second AEC chamber detect is a good indicator of how high the radiation dose is that the lungs 21, 22 are exposed to.

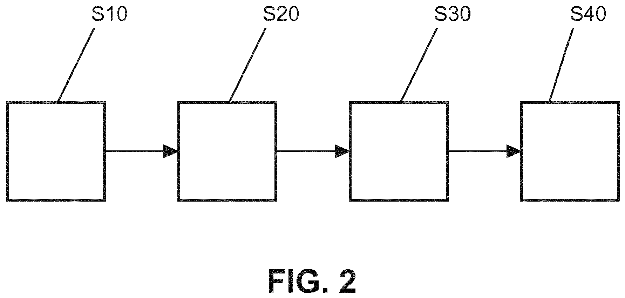

In contrast to the ideal position shown in fig .1, fig. 2 shows a second X-ray image 32 of a left lung 21 and a right lung 22, wherein the patient did not stand in an ideal position between the x-ray source and the first AEC chamber 11 as well as the second AEC chamber 12. The position of the patient to the AEC chamber 11 and the AEC chamber 12 can be determined based on the geometry of the AEC chambers.

Additionally, the second x-ray image 32 is processed by a machine learning algorithm for semantic segmentation of the lungs 21, 22 in the second x-ray image 32. The machines learning algorithm is preferably executed by a deep neural network. Thus, in the second x-ray image 32, the left lung 21 and the right lung 22 can be determined by image recognition. Based on the segmented left lung 21 and the segmented right lung 22 an overlap with the first AEC chamber 11 and the second AEC chamber 12 can be determined. In this case, the overlap ratio between the first AEC chamber 11 and the left lung 21 is below 50%. A reason for such a low overlap can have a plurality of reasons. For example, the patient might be translationally shifted compared to the ideal position between the AEC chamber and the X-ray source. As another example, the whole system of AEC chambers and X-ray source might be miscalibrated. From the overlap ratio itself, in a first step, it can be determined, if the radiation dose detected by the AEC chambers 11, 12 is a reliable source for asserting the radiation dose the patient has been exposed to. Thus, the overlap ratio is compared with a predetermined threshold, which preferably is determined based on the type of medical exam that is performed. In this case, an overlap of under 80% leads to the assumption that the X-ray radiation detected by the AEC chambers was not reliable and that most likely the patient has been exposed to more X-ray radiation than needed. This result can be provided to the operator of the medical exam, for example by a display.

Additionally, from the shape and size of the lungs 21, 22 a root cause for the position deviation between the determined position and the ideal position can be determined. In this case, the left lung 21 is significantly smaller than usual on the left side. This can be the consequence of a translational shift of the patient between the AEC chamber and the X-ray source. Thus, the part of the left lung 21 that should overlap the first AEC chamber is covered by the scapula of the patient, blocking the X-ray radiation between the left lung 21 and the first AEC chamber 11.

In order to provide information about the insufficient overlap of the AEC chambers 11, 12 with the lungs 21, 22, the position of the patient, as determined from the X-ray image, is compared to the ideal position, as predetermined dependent on the performed medical exam. For an improved analysis, not only the single X-ray image 32 needs to be analyzed. Thus, for a plurality of medical exams, a position deviation of the AEC chambers 11, 12 to the ideal position of the AEC chambers 11, 12 is determined and each is associated with additional condition data. The condition data may comprise a room, where the medical exam takes place, a time, when the medical exam takes place and/or an identification of an operator, who positions the patient with respect to the AEC chambers 11, 12. For example, one specific operator has the tendencies to translationally shift the patient between the AEC chambers 11, 12 and the X-ray source to the left compared to the ideal position. This is determined by a statistical analysis of a plurality of position deviations associated with a plurality of condition data.

Thus, in an ideal case, a root cause can be predicted dependent on the condition data before executing the medical exam based on the statistical analysis. For example, for a certain operator, who has to identify himself before executing the medical exam, a common root cause for position deviation can be determined and displayed to the operator. For example, the operator is informed that he has the tendencies to translationally shift the patient slightly to the left. Thus, the operator can correct his usual mistake of dispositioning the patient and an overexposure of the patient with X-ray radiation can be prevented.

Fig. 2 shows a schematic diagram of a method for assessing a position of a patient to an AEC chamber 11, 12 for a medical exam wherein a patient is positioned between an X-ray source and the AEC chamber 11, 12. In a first step S10, an X-ray image 32 of at least part of the patient is acquired, wherein the AEC chamber is configured for detecting a radiation dose, which correlates to a radiation dose of the X-ray source. In a second step S20, a position of the AEC chamber 11, 12 is determined with respect to the patient from the acquired X-ray image 32. In a third step, an exam protocol performed on the patient dependent on the medical exam to be performed on the patient is determined and an ideal position of the AEC chamber 11, 12 is determined with respect to the patient dependent on the exam protocol, wherein the ideal position relates to a position of the patient relative to the AEC chamber 11, 12, in which the detected radiation dose is reliable for the medical exam. In a fourth step S40, a position deviation of the position of the AEC chamber from the ideal position of the AEC chambers is determined.

### LIST OF REFERENCE SIGNS:

- 10: X-ray detector
- 11: first AEC chamber
- 12: second AEC chamber
- 13: third AEC chamber
- 14: fourth AEC chamber
- 15: fifth AEC chamber
- 21: left lung
- 22: right lung
- 31: first X-ray image
- 32: second X-ray image
- 40: scapula
- S10: acquiring an X-ray image
- S20: determining a position of the AEC chamber
- S30: determining an exam protocol
- S40: determining a position deviation

## Claims

1. Method for assessing a position of a patient to an automatic exposure control chamber, AEC chamber (11, 12), for a medical exam, wherein
a patient is positioned between an X-ray source and the AEC chamber (11, 12);
comprising the steps:
- acquiring (S10) an X-ray image (32) of at least part of the patient, wherein the AEC chamber is configured for detecting a radiation dose, which correlates to a radiation dose of the X-ray source;
- determining (S20) a position of the AEC chamber (11, 12) with respect to the patient from the acquired X-ray image (32);
- determining (S30) an exam protocol performed on the patient dependent on the medical exam to be performed on the patient and determining an ideal position of the AEC chamber (11, 12) with respect to the patient dependent on the exam protocol, wherein the ideal position relates to a position of the patient relative to the AEC chamber (11, 12), in which the detected radiation dose is reliable for the medical exam; and
- determining (S40) a position deviation of the position of the AEC chamber from the ideal position of the AEC chambers.

2. Method of claim 1, wherein determining the position deviation comprises the steps:
- segmenting at least an anatomical structure (21, 22) of the patient in the X-ray image (32) thereby determining at least one segmented anatomical structure (21, 22); and
- determining the position deviation dependent on the at least one segmented anatomical structure (21, 22).

3. Method of claim 2, wherein
determining the position deviation comprises the steps:
- determining an overlap of the at least one segmented anatomical structure (21, 22) with the AEC chamber (11, 12); and
- determining the position deviation dependent on the determined overlap.

4. Method of any of the claims 2 to 3, wherein
the at least one anatomical structure (21, 22) of the patient in the X-ray image is segmented by a deep learning network.

5. Method of any of the preceding claims, comprising the steps:
- determining deviation evaluation data, indicating the quantity of the deviation, by comparing the position of the AEC chamber (11, 12) with the ideal position of the AEC chamber (11, 12);
- determining condition data, relating to additional conditions of the specific medical exam; and
- determining a root cause for the position deviation dependent on the deviation evaluation data and the condition data.

6. Method of claim 5, wherein
the condition data comprise a room, where the medical exam takes place, a time, when the medical exam takes place and/or an identification of an operator, who positions the patient with respect to the AEC chamber (11, 12).

7. Method of any of the claims 5 or 6, wherein
the root cause is determined using a statistical analysis of the deviation evaluation data of a plurality of medical exams.

8. Method of any of the claims 4 to 6, comprising the step:
displaying the root cause on a graphical user interface for the operator.

9. Method of any of the claims 4 to 7, comprising the steps:
- providing a database of a plurality of root causes associated with a plurality of condition data; and
- predicting a root cause depending on the condition data and the associated root causes before executing the medical exam.

10. Method of any of the preceding claims, wherein
the exam protocol is determined automatically dependent on the medical exam.

11. Method of any of the claims 6 to 10, comprising the step:
providing the position deviation to the operator.

12. Control unit, configured for executing a method for positioning of an automatic exposure control chamber, AEC chamber (11, 12), for a medical exam.

13. X-ray system, comprising:
- an X-ray source, configured for radiating a patient for a medical exam with X-ray radiation;
- an automatic exposure control chamber, AEC chamber, configured for detecting a radiation dose, which correlates to a radiation dose of the X-ray source; and
- a control unit of claim 12.

14. A computer program, comprising instructions which, when the program is executed by a computer, cause the computer to carry out the method of claims 1 to 11.

15. A computer-readable data carrier having stored there on the computer program of claim 14.
